(19) Europäisches Patentamt

European Patent Office

Office européen des brevets

(11) **EP 1 486 166 B1**

(12) **FASCICULE DE BREVET EUROPEEN**

(45) Date de publication et mention
de la délivrance du brevet:
**30.11.2005 Bulletin 2005/48**

(51) Int Cl.⁷: **A61B 5/0452**

(21) Numéro de dépôt: **04291446.5**

(22) Date de dépôt: **10.06.2004**

(54) **Dispositif d'analyse de l'alternance cycle-à-cycle et/ou de la variabilité de l'onde de repolarisation ventriculaire dans un signal ECG**

Vorrichtung zur Analyse der Zyklus/Zyklus-Alternanz und/oder der Variabilität der ventrikulären Repolarisierungswelle eines EKG-Signals

Device for analysing the cycle-to-cycle alternans and/or the variability of the repolarisation wave of an ECG signal

(84) Etats contractants désignés:
**AT BE BG CH CY CZ DE DK EE ES FI FR GB GR HU IE IT LI LU MC NL PL PT RO SE SI SK TR**

(30) Priorité: **10.06.2003 FR 0306913**

(43) Date de publication de la demande:
**15.12.2004 Bulletin 2004/51**

(73) Titulaire: **ELA MEDICAL**
**92541 Montrouge (FR)**

(72) Inventeurs:
• **Couderc, Jean-Philippe**
**Pittsford 14534 New York (US)**
• **Zareba, Wojciech**
**Rochester 14620 New York (US)**

(74) Mandataire: **Dupuis-Latour, Dominique et al**
**SEP Pagenberg & Associés**
**14 boulevard Malesherbes**
**75008 Paris (FR)**

(56) Documents cités:
**US-A- 5 348 020**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

L'invention concerne un dispositif d'analyse du rythme cardiaque, plus particulièrement pour l'analyse de l'alternance cycle-à-cycle et/ou de la variabilité de l'onde de repolarisation ventriculaire dans un signal ECG.

Un signal ECG, qu'il soit recueilli par des électrodes externes ou par une sonde endocavitaire, présente de façon caractéristique une série de complexes PQRST correspondant à la succession des battements cardiaques du patient.

Sur un cycle cardiaque, le QRS, qui traduit la dépolarisation des ventricules, et suivie par une onde dite "onde T" ou 'onde de repolarisation" (ces deux termes seront utilisés indifféremment par la suite), qui est la traduction électrique sur l'ECG de la repolarisation des cellules myocardiques des ventricules.

L'onde T (onde de repolarisation) présente une amplitude et une forme très variables, et elle est très sensible aux perturbations de la conduction dans le myocarde.

Divers dispositifs ont déjà été proposés pour analyser spécifiquement la variabilité de cette onde T, par exemple le FR-A-2 784 035 (Ela Médical), qui analyse l'onde T pour diagnostiquer l'apparition d'un état ischémique puis l'évolution de cet état en temps réel, de manière à pouvoir adapter en conséquence le fonctionnement du dispositif.

Dans ce document, le dispositif d'analyse de l'onde T est un dispositif incorporé à un implant (stimulateur cardiaque ou cardioverteur/défibrillateur) et opérant en temps réel, par analyse de l'instant d'arrivée du front de repolarisation.

Un autre paramètre de l'onde T qu'il peut être intéressant d'évaluer est l'alternance (*alternans*), qui est une très légère variation répétitive, de l'ordre du millivolt, d'un battement au suivant, de la forme d'onde de l'ECG dans le segment temporel correspondant à l'onde de repolarisation. Cette variation est du type ABABAB..., c'est-à-dire que si l'on examine une onde sur deux, ces ondes sont très semblables, mais qu'en revanche d'une onde à l'onde immédiatement suivante peut apparaître une variation détectable de l'amplitude, dont le niveau constitue un indicateur important d'une instabilité électrique cardiaque du patient.

La présence d'une alternance de l'onde T, révélatrice d'une repolarisation non uniforme du myocarde, est en particulier un très bon prédicteur de fibrillation, et donc du risque clinique d'arythmie ventriculaire et de mort subite.

Dans le cas d'un défibrillateur/cardioverteur implanté avec un analyseur intégré d'alternance d'onde T, comme décrit par exemple par le FR-A-2 808 213 (Medtronic Inc.), il est possible de fournir rapidement un avertissement au patient ou au médecin en cas de risque de risque cardiaque majeur, ou même de déclencher une thérapie par le dispositif lorsque ce risque est avéré.

Indépendamment des dispositifs implantés intégrant un analyseur fonctionnant en temps réel, il est également possible de rechercher la présence d'une alternance d'onde T à partir de signaux recueillis par un enregistreur dit "Holter", c'est-à-dire un appareil réalisant en continu et sur une longue période l'enregistrement de signaux recueillis au moyen d'électrodes implantées ou d'électrodes externes. Le dépouillement de l'enregistrement Holter peut alors inclure la recherche d'une éventuelle alternance de l'onde T, constituant un marqueur de risque. Une telle analyse prend toute son importance dans la définition des patients susceptibles de bénéficier au mieux de l'implantation d'un défibrillateur / cardioverteur implantable en prévention primaire.

La présence d'une alternance de l'onde T est également un prédicteur important d'une dégradation de l'état ischémique du patient. En effet, un état ischémique se traduit par une modification quasi-instantanée et détectable de l'onde de repolarisation ventriculaire (l'ischémie apparaît à la suite d'un arrêt ou d'une réduction de l'irrigation sanguine du coeur).

La recherche d'un alternance de l'onde T s'est jusqu'à présent révélée relativement difficile car la variation cycle-à-cycle révélatrice de cette altemance est très faible (typiquement une variation de l'ordre de 5 µV) notamment par rapport au niveau moyen du bruit présent dans le signal ECG, ce bruit ayant un niveau typique de l'ordre de 10 µV. La recherche d'une alternance de l'onde T nécessite donc la mise en oeuvre de moyens qui à la fois soient très sensibles et présentent une bonne immunité au bruit (impliquant donc des algorithmes et des filtrages complexes).

De ce fait, les algorithmes proposés jusqu'à présent nécessitaient des moyens de calcul relativement importants, impliquant donc des ressources de calcul ne permettant pas leur mise en oeuvre dans un micro-ordinateur ou, à plus forte raison, dans un appareil ambulatoire ou implanté, si ce n'est au prix d'un temps de traitement excessif et/ou d'une moindre qualité du résultat. Or, pour obtenir rapidement un prédicteur fiable de fibrillation ou d'ischémie du myocarde, il est important de pouvoir révéler et discriminer rapidement un certain nombre de microvariations, qui peuvent être très significatives pour l'obtention d'un diagnostic fiable et pertinent.

Parmi les diverses techniques d'analyse de l'alternance de l'onde de T proposées jusqu'à présent, on peut citer :

- la méthode spectrale (voir notamment US-A-4 802 491 et Rosembaum DS et al., Electrical Alternans and Vulnerability to Ventricular Arrhythmias. *N Engl J Med* 1994;*330*:235-241) : cette technique propose d'analyser les variations énergétiques du spectre fréquentiel du signal ECG, de manière à rechercher un pic d'énergie pour une fréquence révélatrice de la fluctuation recherchée ;

- la technique de démodulation complexe (voir US-A-5 842 997 et Nearing B et al., Dynamic Tracking of Cardiac Vulnerability by Complex Demodulation of the T-Wave, *Science* 1991;*252*:437-440) : cette technique cherche à modéliser la fluctuation de l'amplitude de l'onde T par une sinusoïde d'amplitude et de phase variables, de manière à assurer un suivi dynamique des variations d'alternance de l'onde T ; sa complexité intrinsèque rend cependant la méthode difficile à appliquer sans ajout de circuits matériels spécifiques ;

- la technique d'analyse dans le domaine temporel (voir Verrier R. et al., Médian Beat Analysis of T-Wave Alternans to Predict Arrhythmic Death after Myocardial Infarction: Results from the Autonomic Tone and Reflexes after Myocardial Infarction Study, *Circulation,* 102, 18;2000: II-713 (abstract)) : cette méthode consiste à calculer, un battement sur deux, la moyenne du niveau de l'onde T en un point donné du segment de repolarisation et à quantifier la différence d'amplitude entre les deux moyennes ;

- la technique d'étirement (voir US-A-5 560 638 et Berger R et al., Beat-to-Beat QT Interval Variability: Novel Evidence for Repolarization Ability in Ischemic and Non-Ischemic Dilated Cardiomyopathy, *Circulation* 1997;*96*: 1557-1565): dans cette technique, on superpose l'onde T à un gabarit et la composante temporelle est étirée de manière à minimiser la différence entre le gabarit et le battement analysé ;

- la technique par intercorrélation (voir Burratini L et al., Computer Detection of Non-Stationary T-Wave Alternans Using New Correlative Method, Computers in Cardiology 1997;*42*:657-660) : il s'agit d'une technique consistant à quantifier, dans le domaine temporel, les variations d'amplitude et de morphologie de l'onde de repolarisation sur la base d'un indice de corrélation ; chaque onde T est corrélée avec une onde T moyenne représentative d'une série de battements, une alternance, positive ou négative, se traduisant par une oscillation de l'indice de corrélation autour de la valeur unité ;

- l'approche par ondelettes (voir Couderc JP et al., Beat-to-Beat Repolarization Variability in LQTS Patients with the SCN5A Sodium Channel Gene Mutation, *PACE* 1999, 22, 1581-1592) : le signal ECG est décomposé en une somme de gaussiennes puis traité de manière à isoler les différentes composantes de l'onde (P, QRS et T) et faire apparaître ainsi des singularités, révélatrices notamment d'une alternance pour l'onde T.

La présente invention a pour but de proposer une nouvelle technique de détection et de quantification de la variabilité et de l'alternance de l'onde T dans un signal ECG.

Par rapport aux techniques antérieures évoquées ci-dessus, la technique de l'invention :

- procède dans le domaine temporel pour évaluer la variabilité ou l'alternance de l'onde de repolarisation, évitant ainsi le recours à des transformations de Fourier ou autres techniques d'analyse spectrale nécessitant des moyens de calcul importants ;

- ne se base pas sur une méthode de corrélation des signaux pour la synchronisation et la quantification de la variabilité et de l'alternance de l'onde T ;

- permet le suivi d'une alternance ou d'une variabilité de la repolarisation dans n'importe quel signal ECG présentant une résolution en amplitude et une fréquence d'échantillonnage suffisantes, et est donc utilisable avec n'importe quel appareil existant, sur la base d'enregistrements effectués selon des techniques classiques et éprouvées, sans requérir aucun appareil dédié ni adaptation matérielle des dispositifs existants ;

- permet d'identifier une partie du segment de repolarisation où la variabilité et/ou l'alternance sont maximales, augmentant ainsi la pertinence et la sélectivité de la mesure.

Le dispositif d'analyse de l'invention opère sur un signal ECG préalablement recueilli par un dispositif médical implanté (stimulateur cardiaque disposant de fonctions Holter, défibrillateur/cardioverteur ou dispositif multi sites) ou externe (enregistreur Holter ambulatoire), puis filtré, échantillonné et numérisé de manière en elle-même connue.

De façon caractéristique de l'invention, le dispositif d'analyse de l'alternance cycle-à-cycle et/ou de la variabilité de l'onde de repolarisation ventriculaire dans le signal ECG comprend :

- des moyens extracteurs, aptes à extraire du signal ECG, pour chaque battement cardiaque, un segment temporel de $T$ échantillons de l'onde de repolarisation ventriculaire, considéré à partir d'un instant $t$ suivant une origine temporelle prédéterminée ;

- des moyens de mémorisation, aptes à mémoriser les $T$ échantillons ainsi extraits, pour $B$ battements consécutifs considérés à partir du $b^{\text{ième}}$ battement du signal ECG, de manière à sélectionner et mémoriser un agrégat bidimensionnel de Tx B échantillons de signal contigus dans l'espace temps-battements ;

- des moyens évaluateurs, aptes à calculer un facteur de variance locale de l'onde de repolarisation ventriculaire, représentatif d'une mesure de la variance du niveau de signal des échantillons à l'intérieur de l'agrégat ; et

- des moyens pondérateurs, aptes à :

- détecter et comptabiliser les alternances cycle-à-cycle de l'onde de repolarisation ventriculaire sur les *B* battements de l'agrégat,
- pondérer le facteur de variance locale par un facteur d'alternance locale variant entre un minimum, correspondant à l'absence complète de détection d'alternance sur les battements de l'agrégat, et un maximum, correspondant à la détection d'une alternance permanente et récurrente sur tous les battements de l'agrégat, et
- délivrer en sortie, un indice d'alternance et de variabilité fonction, pour un battement donné, du facteur de variance locale pondéré par le facteur d'alternance locale.

Selon diverses formes de mise en oeuvre avantageuses :

- la pondération par le facteur d'alternance locale est opérée sur la racine carrée du facteur de variance locale ;
- les moyens pondérateurs sont des moyens aptes à calculer le facteur d'alternance locale pour une pluralité prédéterminée de sous-segments temporels de durées et d'origines différentes au sein de l'agrégat, puis sélectionner parmi ces sous-segments celui pour lequel le facteur d'alternance locale correspondant est maximal, et pondérer le facteur de variance locale correspondant au sous-segment ainsi sélectionné par ce facteur d'alternance locale maximal ;
- les moyens pondérateurs sont des moyens aptes à calculer le facteur d'alternance locale par recherche et quantification de la répétition d'un motif d'alternance prédéfini à l'intérieur de l'agrégat, pour une même position de l'échantillon dans le segment temporel. Ce calcul peut notamment être opéré par recherche et comptage des changements de signe consécutifs de la dérivée du signal dans le domaine des battements, pour une même position de l'échantillon dans le segment temporel ;
- le dispositif comporte en outre des moyens de prétraitement du signal avant son application aux moyens extracteurs, ces moyens de prétraitement comprenant au moins l'une des moyens du groupe comprenant : un filtre passe-bas ; un filtre passe-bas à réponse impulsionnelle finie ; un filtre d'élimination de la ligne isoélectrique ; et un filtre d'élimination de la composante respiratoire ;
- le dispositif comporte en outre des moyens sélecteurs d'échantillons, aptes à rechercher dans le signal ECG une séquence de battements présentant, sur toute la durée de *B* battements consécutifs, un rythme cardiaque stable et ne comportant que des cycles d'origine sinusale sauf les cycles générés par un stimulateur cardiaque, et à n'appliquer aux moyens extracteurs qu'une telle séquence d'échantillons ; les moyens sélecteurs peuvent notamment être des moyens aptes à calculer les intervalles RR desdits *B* battements consécutifs, et à définir un rythme cardiaque stable pour cette séquence d'échantillons si aucun de ces intervalles ne varie de plus d'un pourcentage donné, notamment pas plus de 10 %, par rapport à la valeur moyenne de l'intervalle RR calculée sur les B battements.

On va maintenant décrire un exemple de mise en oeuvre du dispositif de l'invention, en référence aux dessins annexés.

La figure 1 est un schéma montrant les étapes successives de traitement du signal aboutissant à la détermination d'un indice de variabilité et d'alternance.
La figure 2 est une représentation dans un espace tridimensionnel des cycles cardiaques successifs, permettant de faire apparaître un agrégat local à partir duquel sera calculé l'indice d'alternance et de variabilité.
Les figures 3a et 3b illustrent la manière de calculer le facteur de variance locale et le facteur d'alternance locale pour l'agrégat isolé de la figure 2.
Les figures 4a et 4b illustrent des affichages des variations de l'indice de variabilité d'alternance, respectivement pour un patient avec alternance de l'onde T documentée et pour un sujet sain.
Les figures 5a et 5b sont des histogrammes illustrant les performances de l'analyse en termes de sensibilité, respectivement pour une technique fréquentielle connue et pour la technique de l'invention.
Les figures 6a et 6b sont des histogrammes illustrant les performances de l'analyse en termes de spécificité, respectivement pour une technique fréquentielle connue et pour la technique de l'invention.
Les figures 7a et 7b montrent la variation au cours du temps de l'indice de variabilité d'alternance dans le cas d'un suivi en temps réel, respectivement pour un sujet sain et pour un patient avec alternance de l'onde T.

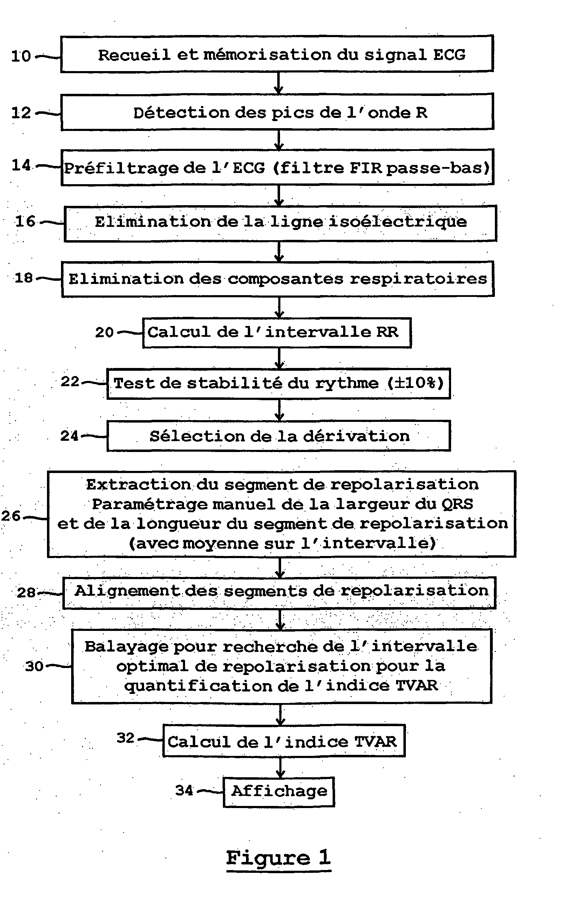

La figure 1 illustre les différentes étapes de traitement du signal.
Tout d'abord (étape 10), le signal ECG est recueilli et mémorisé sous forme numérique d'une succession d'échantillons d'amplitude variable. Comme indiqué plus haut, le signal ECG est recueilli par un dispositif médical implanté (stimulateur cardiaque disposant de fonctions Holter, défibrillateur/cardioverteur ou dispositif multi sites) ou externe (enregistreur Holter ambulatoire), puis filtré, échantillonné et numérisé de manière en elle-même connue.
L'étape suivante (étape 12) consiste à détecter les pics de l'onde R du signal ECG, de manière à individualiser les battements successifs constituant le signal ECG.

Le signal fait ensuite l'objet d'un préfiltrage (étape 14), avantageusement un filtrage passe-bas par un filtre FIR (filtre à réponse impulsionnelle finie, qui est un filtre numérique non récursif).

La composante continue, correspondant à la ligne isoélectrique, est ensuite éliminée (étape 16), ainsi que les composantes respiratoires, qui se traduisent par des variations lentes du niveau moyen du signal (étape 18). Le dispositif calcule ensuite la valeur de l'intervalle RR sur une série de battements successifs (étape 20), de manière à ne poursuivre l'analyse du segment de repolarisation que dans des conditions de rythme cardiaque stable, la stabilité étant par exemple définie par une variation de l'intervalle RR inférieure à 10 % (étape 22).

Dans le cas d'un ECG de surface, le dispositif sélectionne l'une des dérivations (étape 24), par exemple dérivation VM, A, B ou C pour un dispositif à 4 voies. D'autres dispositifs (typiquement à 2, 3, 5, 9 ou 12 voies) peuvent être utilisés, la sélection portant alors sur l'une des voies 1 à 12, ou A à L.

Pour chaque battement, le dispositif extrait ensuite le segment de repolarisation sur lequel l'analyse sera spécifiquement opérée ; un certain nombre de paramètres peuvent être fixés manuellement à cet effet, notamment : (i) la largeur du QRS ; (ii) la longueur du segment de repolarisation ; et (iii) le nombre d'échantillons du signal considérés pour le découpage de l'onde T, paramètre indispensable pour l'identification de la portion de l'onde T dans laquelle l'alternance et/ou la variabilité sont maximales (étape 26).

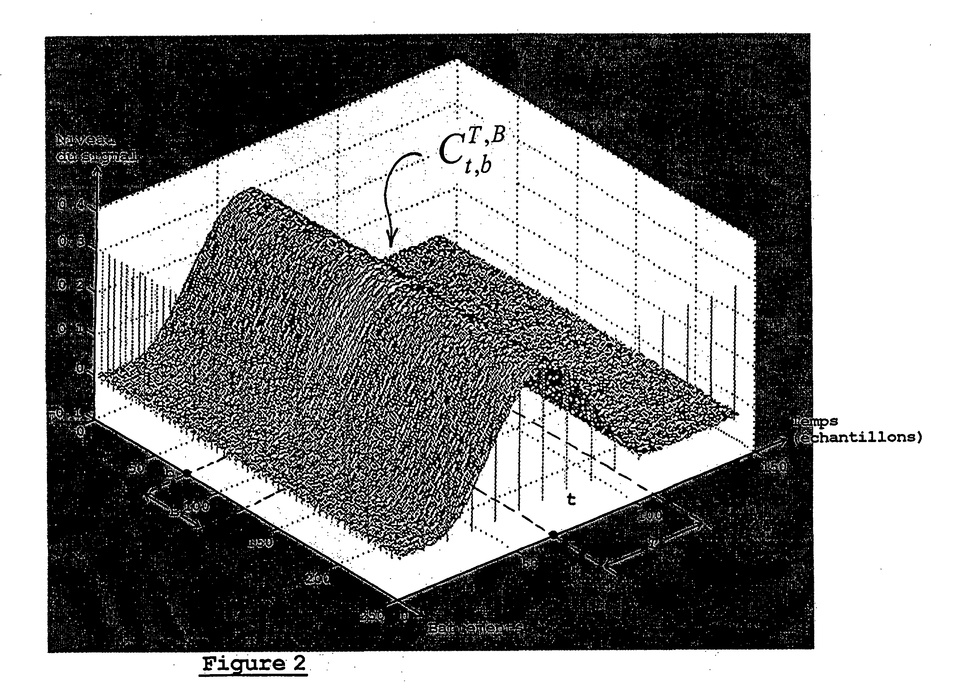

Les segments de repolarisation ainsi individualisés sont alignés côte à côte, de la manière que l'on expliquera plus en détail en référence à la figure 2 (étape 28).

Les données ainsi regroupées sont balayées de manière à rechercher l'intervalle optimal du segment de repolarisation pour la quantification de l'indice de variabilité et d'alternance de l'onde T (étape 30).

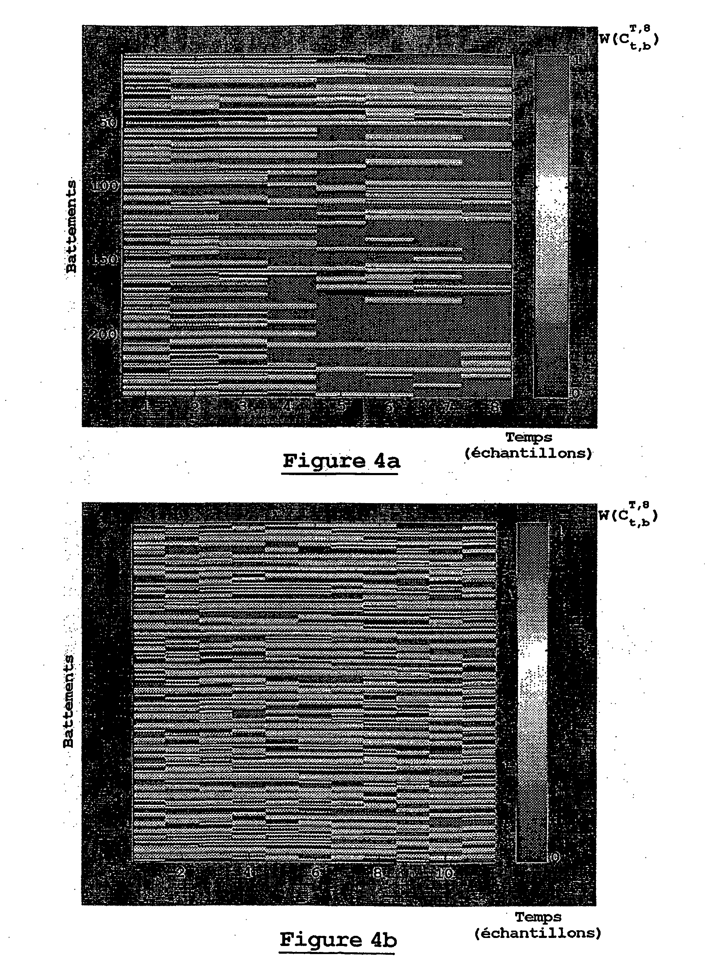

Cet indice est ensuite calculé (étape 32) de la manière que l'on exposera plus bas, puis affiché (étape 34), par exemple de la manière montrée sur les figures 4a et 4b.

La technique de l'invention vise ainsi à extraire du signal ECG un indice représentatif de la variabilité de l'onde T dans ce signal ECG, ou plus généralement de l'alternance et de la variabilité de cette onde T, indice que l'on désignera par la suite "indice TVar".

Essentiellement, la méthode mise en oeuvre par le dispositif de l'invention consiste à réaliser une analyse bidimensionnelle, à la fois dans le domaine temporel et dans le domaine des battements successifs :

- l'utilisation du domaine temporel pour l'étude de la variabilité et de l'alternance de l'onde T présente l'avantage d'un bon compromis entre les résolutions temporelles et d'amplitude du signal numérisé ainsi que, comme on le verra plus bas, la possibilité d'identifier et d'isoler la partie la plus significative de l'onde T pour la quantification de la variabilité et de l'alternance ;
- quant à l'utilisation du domaine des battements, elle permet de mieux identifier des phénomènes transitoires de variabilité et d'alternance dans le cas de battements non sinusaux, par exemple pendant de brèves périodes d'exercice au cours de l'activité quotidienne du porteur d'un enregistrement Holter.

La figure 2 est une représentation tridimensionnelle des ondes T respectives des battements successifs du signal ECG, ces ondes T ayant été isolées dans chaque battement et juxtaposées de manière à en donner une représentation montrant leur évolution au cours des battements successifs.

La représentation de la figure 2 est donnée avec :

- en abscisse : le temps, compté en nombre d'échantillons à partir d'une origine correspondant à un instant d'arrivée de l'onde de repolarisation déterminée de manière identique d'un battement au suivant (cet instant d'arrivée de l'onde de repolarisation peut être par exemple déterminé de la manière décrite dans le FR-A-2 784 035 précité) ;
- en ordonnée : les battements, comptés en nombre de battements depuis une origine donnée à partir de laquelle on a détecté une succession ininterrompue de battements présentant un rythme cardiaque (intervalle RR) stable ;
- en cote : le niveau du signal, en microvolts (valeur numérisée).

La première étape consiste à isoler, dans l'espace bidimensionnel temps-battements, un agrégat d'échantillons, désigné $C_{t,b}^{T,B}$, constitué de $B$ x T échantillons :

- situés sur un segment de longueur $T$ et d'origine temporelle $t$ de chaque onde de repolarisation,
- et ceci pour $B$ battements successifs, comptés à partir du $b$[ième] battement.

On peut ainsi par exemple isoler un agrégat $C_{t,b}^{20,16}$ pour un même segment de $T$ = 20 échantillons, considérés sur $B$ = 16 battements successifs.

À partir de cet agrégat d'échantillons, le dispositif de l'invention va calculer un indice, désigné $TVar_b$, donnant une mesure de la variabilité de l'onde T en une position temporelle $t$ de cette onde, cette mesure étant pondérée par un facteur représentatif de la survenue, plus ou moins récurrente, d'une alternance.

En d'autres termes, l'indice $TVar_b$ est une mesure d'une combinaison de la variabilité de l'onde T et de l'alternance de l'onde T.

Cet indice $TVar_b$ peut s'exprimer sous la forme :

$$TVar_b = \sqrt{Var(C_{to,b}^{\overline{Tm,B}})} \times Max_{Tm,to}(W(C_{t,b}^{T,B}))$$

Le premier terme,

$$\sqrt{Var(C_{t,b}^{\overline{Tm,B}})},$$

de cette expression est la racine carrée de la variance locale, fonction qui donne une mesure de la variabilité du signal au sein de l'agrégat dans la région ou l'alternance est maximale (on verra plus loin la manière dont on détermine cette région d'alternance maximale).

Le second terme de l'expression inclut un facteur $W(C_{t,b}^{T,B})$ d'alternance locale dont la valeur varie de 0 à 1 ($0 \leq W(C_{t,b}^{T,B}) \leq 1$ avec $W(C_{t,b}^{T,B}) = 1$ en cas d'alternance permanente, parfaitement récurrente sur la totalité des *B* battements de l'agrégat et, inversement, $W(C_{t,b}^{T,B}) = 0$ en l'absence totale d'alternance sur ces *B* battements.

Le facteur d'alternance locale $W(C_{t,b}^{T,B})$ est calculé pour différentes longueurs *T* du secteur de repolarisation et pour différentes origines temporelles *t* sur ce même segment, c'est-à-dire, en d'autres termes, pour un agrégat $C_{t,b}^{T,B}$ où un algorithme de balayage fait varier les paramètres *t* et *T* dans des limites fixées au préalable, par exemple par un paramétrage manuel.

Cette recherche d'un maximum donnera des valeurs *Tm* et *to,* correspondant donc, respectivement, à la longueur et à l'origine temporelle du segment de repolarisation pour lequel le facteur d'alternance locale, c'est-à-dire $C_{to,b}^{Tm,B}$ est maximal.

La variance locale $Var(C_{t,b}^{Tm,B})$ est évaluée pour l'agrégat ainsi isolé.

Le facteur d'alternance locale $W(C_{t,b}^{T,B})$ correspondant lui est appliqué en tant que facteur de pondération, donnant ainsi pour chaque battement *b* un indice de variabilité et d'alternance $TVar_b$ :

$$TVar_b = \sqrt{Var(C_{to,b}^{\overline{Tm,B}})} \times Max_{Tm,to} W(C_{t,b}^{T,B})).$$

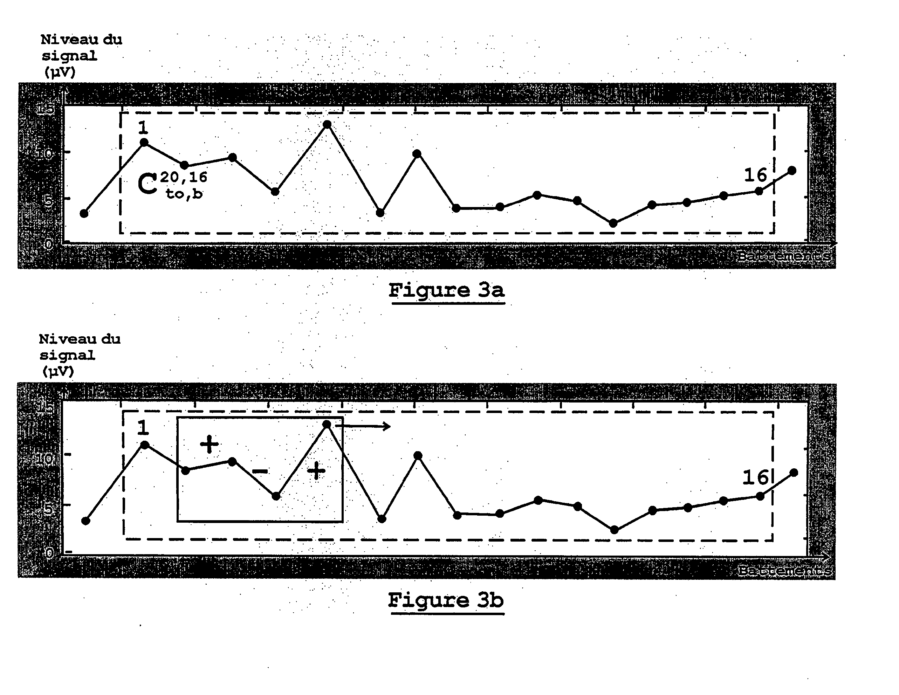

On va maintenant indiquer plus précisément, en référence aux figures 3a et 3b, la manière dont peuvent être calculés le facteur de variance locale $Var(C_{t,b}^{Tm,B})$ et le facteur d'alternance locale $W(C_{t,b}^{T,B})$.

La variance locale $Var(C_{t,b}^{Tm,B})$ est obtenue par un calcul classique de variance, soit :

$$Var(C_{to,b}^{Tm,B}) = \frac{1}{B} \left( \sum_{n=b}^{b+B-1} (S_{t_o}^{Tm}(n) - \overline{S_{t_o}^{Tm}})^2 \right),$$

avec:

$$S_{t_0}^{Tm}(n) = \frac{1}{Tm} \sum_{k=to}^{to+Tm-1} S_k(n),$$

et

$$\overline{S_{t_o}^{Tm}} = \frac{1}{B} \sum_{k=b}^{b+B-1} S_{t_o}^{Tm}(k),$$

*T* = *Tm* représentant la durée optimale assurant la valeur la plus élevée de la fonction de pondération (alternance locale),

$S_k(n)$ étant l'amplitude du segment de repolarisation pour le battement $n$ et l'échantillon $k$,

$S_{t_o}^{Tm}(n)$ étant l'amplitude moyenne de la repolarisation dans l'agrégat situé à l'échantillon $to$ et comprenant $Tm$ échantillons, pour un battement $n$ donné, et

$\overline{S_{t}^{Tm}}$ étant l'amplitude moyenne de $S_{t}^{Tm}(n)$ sur les $B$ battements compris dans l'agrégat.

Ainsi, par exemple, sur la base d'un agrégat de $Bx$ $Tm$ échantillons commençant par exemple à l'instant $t =$ 20 à partir du $b = 16^e$ battement :

$$\text{Var}(C_{to,b}^{20,16}) = \frac{1}{16}\left(\sum_{n=b}^{b+15}(S_{t_o}^{20}(n) - \overline{S_{t_o}^{20}})^2\right),$$

c'est-à-dire la variance du $20^e$ échantillon de l'onde T, considérée sur une fenêtre glissante (représentée en tiretés sur la figure 3a) de 16 battements successifs.

On va maintenant indiquer plus précisément, en référence à la figure 3b, la manière dont est calculé le facteur d'alternance locale $W(C_{t,b}^{T,B})$.

Le dispositif recherche, sur les battements successifs, le plus petit motif élémentaire d'alternance c'est-à-dire, comme indiqué dans le cadre en trait plein sur la figure 3b, un motif de quatre battements successifs présentant trois inversions de signes, de type "+ - +" ou "- + -". Dans l'exemple illustré, le motif est du type "+ - +", c'est-à-dire que, d'un battement au suivant, le niveau augmente, puis diminue, puis augmente à nouveau.

Une fois fixé le nombre de battements de l'agrégat, le dispositif examine la variation cycle-à-cycle du niveau de signal afin de déterminer la survenue (présence d'un motif "+ - +" ou "- + -"), ou non, d'un motif d'alternance et détermine la proportion, entre 0 et 100 % (facteur $W(C_{t,b}^{T,B})$) de survenue de ce motif sur la série de battements.

Plus précisément, ce facteur $W(C_{t,b}^{T,B})$ peut être déterminé numériquement sous la forme :

$$W(C_{t,b}^{T,B}) = \frac{1}{(b-k)}\sum_{n=B}^{B+b-k-1}ALT_n.$$

La fonction $ALT_n$ est une fonction assurant le comptage du nombre de motifs de base d'alternance à l'intérieur d'un agrégat donné, avec :

$$ALT_n = \frac{1}{2(k-1)}\sum_{l=n}^{n+k-2}\left|\frac{d}{db}\left(sign\left(\frac{d(S_l - \min(S))}{db}\right)\right)\right|.$$

Ainsi, $ALT_n$ vaut 1 si tous les battements suivant le battement $n$ présentent une alternance.

La fonction de pondération (facteur d'alternance locale) $W(C_{t,b}^{T,B})$ pour un agrégat $C_{t,b}^{T,B}$ donné est représenté par la valeur moyenne de la survenue des motifs d'alternance de base à l'intérieur de cet agrégat.

On va maintenant donner, en référence aux figures 4a et 4b, des exemples montrant la manière dont ces paramètres peuvent varier dans l'espace temps/battements, respectivement pour un patient présentant une alternance de l'onde T avérée (figure 4a), et pour un patient ne présentant pas ce symptôme (figure 4b).

La figure 4a et la figure 4b montrent la distribution des valeurs du facteur de pondération $W(C_{t,b}^{T,B})$ avec :

- en abscisse : la position temporelle de l'agrégat à l'intérieur de l'onde T (par exemple, l'agrégat n° 1 correspond aux premières 40 ms de l'onde T, l'agrégat n° 2 se situe entre 40 et 80 ms à l'intérieur de l'onde T, etc.),
- en ordonnée : la succession des battements, et
- en cote : l'amplitude, entre 0 et 1, du facteur d'alternance (facteur de pondération $W(C_{t,b}^{T,B})$), sur la base d'une échelle en fausses couleurs (échelle de gris).

On voit très nettement, chez le patient présentant une alternance d'onde T documentée, des coefficients W

($C_{t,b}^{T,B}$) significativement élevés révélant une alternance située dans les derniers agrégats du segment (centre et partie droite de la figure 4a). Au contraire, chez un patient sans alternance, les valeurs de $W(C_{t,b}^{T,B})$ sont beaucoup plus dispersées sur toute l'étendue du segment et la pondération par l'alternance locale est dans l'ensemble beaucoup plus faible (figure 4b).

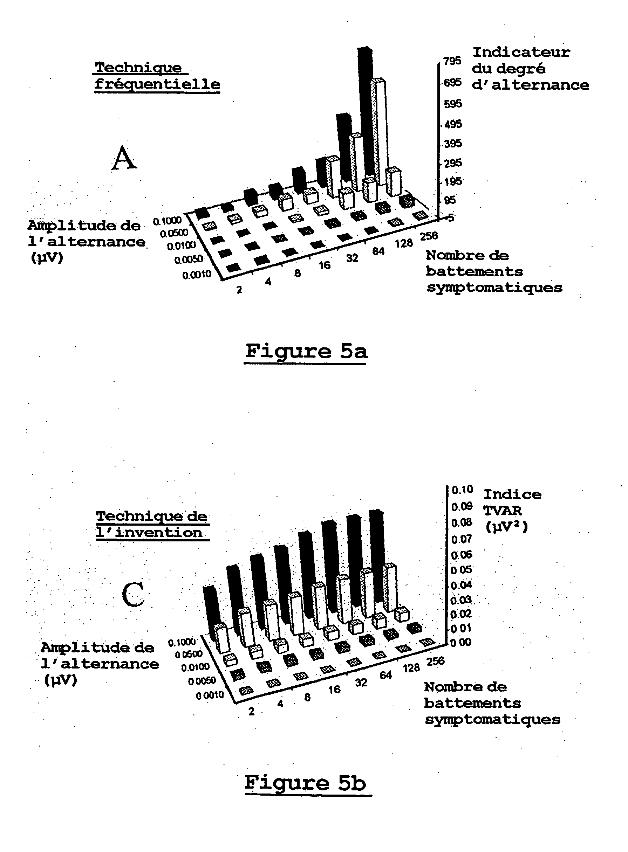

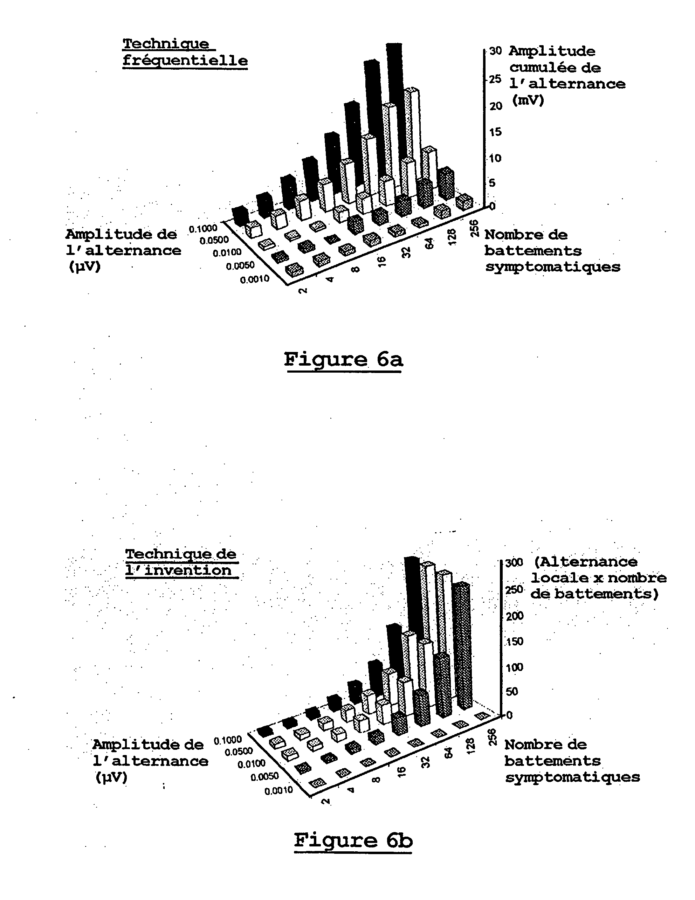

Les figures 5a, 5b, 6a et 6b sont des histogrammes tridimensionnels réalisés sur la base de simulations, respectivement pour une technique fréquentielle classique (figures 5a et 6a) et par la technique mise en oeuvre par le dispositif de l'invention (figures 5b et 6b).

Ces figures sont des histogrammes donnant les valeurs de divers paramètres pour un signal simulé avec, en abscisse, un nombre croissant de battements présentant une alternance et, en ordonnée, une amplitude croissante de cette alternance cycle-à-cycle.

Les paramètres illustrés sont les suivants :

- figure 5a (technique fréquentielle, connue) : taux d'alternance, donnant une estimation statistique du niveau de l'alternance dans le signal ;
- figure 5b (technique de l'invention) : estimation du niveau de l'alternance à partir de l'indice TVar ;
- figure 6a (technique fréquentielle, connue) : tension d'alternance cumulée, donnant une estimation de l'alternance cumulativement sur la totalité du signal ;
- figure 6b (technique de invention) : nombre de battements présentant une alternance, obtenu par le produit du facteur d'alternance locale $W(C_{t,b}^{T,B})$ par le nombre de battements du signal.

La comparaison des figures 5a et 5b montre en particulier une sensibilité notablement plus élevée lorsqu'il s'agit de détecter des alternances de faible amplitude.

Ces figures montrent également la supériorité de la technique de l'invention, par rapport à la technique connue, pour détecter la survenue d'alternances présentes sur un faible nombre de battements seulement.

Les figures 6a et 6b confirment les constatations précédentes, cette fois à l'égard de l'évaluation du nombre de battements symptomatiques (par la technique de l'invention) et de l'estimation de la tension d'alternance (par la technique fréquentielle connue). Ici encore, à l'exception des alternances de très faible amplitude, on constate que la technique de l'invention permet une meilleure estimation du nombre de battements symptomatiques lorsque seul un faible nombre de battements présente une alternance.

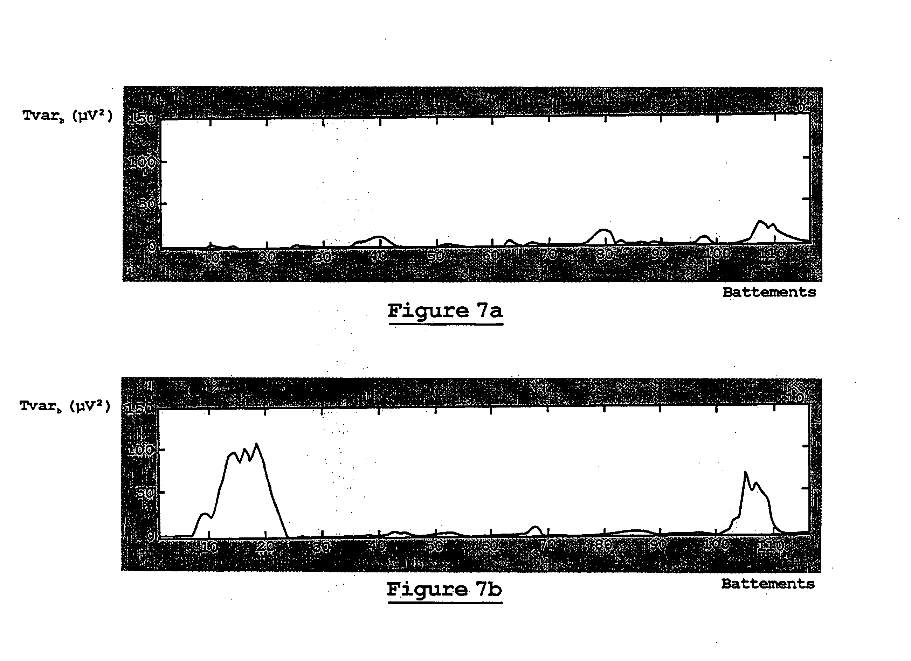

Les figures 7a et 7b, quant à elles, illustrent des résultats cliniques obtenus, respectivement sur un sujet sain (figure 7a) et sur un patient présentant des épisodes d'alternance de l'onde T (figure 7b), lorsque la technique de l'invention est utilisée pour un suivi en continu de l'indice de variabilité et d'alternance TVar. Ces figures illustrent l'évolution du paramètre $TVar_b$ au fil des battements successifs (identifiés par leur position temporelle *b* dans la séquence de battements faisant l'objet du suivi).

La technique de l'invention permet de détecter des épisodes d'alternance de l'onde T mêmes brefs (dans l'exemple illustré, d'une durée de 10 à 15 battements), ce qui permet d'assurer un suivi pratiquement en temps réel de ce paramètre. Il devient ainsi possible d'étudier la fréquence d'apparition d'une alternance sporadique, sa durée moyenne, la corréler à d'autres indicateurs (effort, activité, etc.) de manière à pouvoir fournir au praticien des informations pertinentes en vue d'un possible diagnostic de risque de fibrillation et/ou d'ischémie myocardique. On notera que la technique de l'invention ne produit pas de faux positifs, le niveau de l'indice $TVar_b$ restant toujours quasi-nul au cours du temps chez un patient sain (figure 7a).

**Revendications**

**1.** Un dispositif d'analyse de l'alternance cycle-à-cycle et/ou de la variabilité de l'onde de repolarisation ventriculaire dans un signal ECG comprenant une série de battements cardiaques, ce signal ayant été préalablement recueilli par un dispositif médical puis filtré, échantillonné et numérisé,
ce dispositif d'analyse étant **caractérisé par** :

- des moyens extracteurs, aptes à extraire du signal ECG, pour chaque battement cardiaque, un segment temporel de *T* échantillons de l'onde de repolarisation ventriculaire, considéré à partir d'un instant *t* suivant une origine temporelle prédéterminée ;
- des moyens de mémorisation, aptes à mémoriser les *T* échantillons ainsi extraits, pour B battements consécutifs considérés à partir du *b*-ième battement du signal ECG, de manière à sélectionner et mémoriser un agrégat bidimensionnel ($C_{t,b}^{Tm,B}$) de *TxB* échantillons de signal contigus dans l'espace temps-battements ;
- des moyens évaluateurs, aptes à calculer un facteur de variance locale de l'onde de repolarisation ventriculaire ($Var(C_{t,b}^{Tm,B})$), représentatif d'une mesure de la variance du niveau de signal des échantillons à l'intérieur de l'agrégat ; et

- des moyens pondérateurs, aptes à :

  • détecter et comptabiliser les alternances cycle-à-cycle de l'onde de repolarisation ventriculaire sur les *B* battements de l'agrégat,
  • pondérer le facteur de variance locale ( $\mathrm{Var}(C_{t,b}^{Tm,B})$ ) par un facteur d'alternance locale ($W(C_{t,b}^{T,B})$) variant entre un minimum (W = 0), correspondant à l'absence complète de détection d'alternance sur les battements de l'agrégat, et un maximum (W = 1), correspondant à la détection d'une alternance permanente et récurrente sur tous les battements de l'agrégat, et
  • délivrer en sortie, un indice d'alternance et de variabilité ($TVar_b$) fonction, pour un battement donné, du facteur de variance locale pondéré par le facteur d'alternance locale.

2. Le dispositif de la revendication 1, dans lequel les moyens pondérateurs sont des moyens aptes à :

   a) calculer le facteur d'alternance locale ($W(C_{t,b}^{T,B})$) pour une pluralité prédéterminée de sous-segments temporels de durées et d'origines différentes au sein de l'agrégat, puis
   b) sélectionner parmi ces sous-segments celui ($T_m$, $t_0$) pour lequel le facteur d'alternance locale correspondant ($W(C_{to,b}^{Tm,B})$) est maximal, et
   c) pondérer le facteur de variance locale ($\mathrm{Var}(C_{to,b}^{Tm,B})$) correspondant au sous-segment ainsi sélectionné par ce facteur d'alternance locale maximal ($\mathrm{Max}_{Tm,to}(W(C_{t,b\;t,b}^{T,B\;T,B}))$).

3. Le dispositif de la revendication 1 ou 2, dans lequel la pondération par le facteur d'alternance locale est opérée sur la racine carrée du facteur de variance locale ($\mathrm{Var}(C_{t,b}^{Tm,B})$).

4. Le dispositif de la revendication 1, dans lequel les moyens pondérateurs sont des moyens aptes à calculer le facteur d'alternance locale par recherche et quantification de la répétition d'un motif d'alternance prédéfini à l'intérieur de l'agrégat, pour une même position de l'échantillon dans le segment temporel.

5. Le dispositif de la revendication 4, dans lequel les moyens pondérateurs sont des moyens aptes à calculer le facteur d'alternance locale par recherche et comptage des changements de signe consécutifs de la dérivée du signal dans le domaine des battements, pour une même position de l'échantillon dans le segment temporel.

6. Le dispositif de la revendication 1, comportant en outre des moyens de prétraitement du signal avant son application aux moyens extracteurs, ces moyens de prétraitement comprenant au moins l'une des moyens du groupe comprenant : un filtre passe-bas (14) ; un filtre passe-bas à réponse impulsionnelle finie (14) ; un filtre d'élimination de la ligne isoélectrique (16) ; et un filtre d'élimination de la composante respiratoire (18).

7. Le dispositif de la revendication 1, comportant en outre des moyens sélecteurs d'échantillons (12, 20, 22), aptes à rechercher dans le signal ECG une séquence de battements présentant, sur toute la durée de *B* battements consécutifs, un rythme cardiaque stable et ne comportant que des cycles d'origine sinusale sauf les cycles générés par un stimulateur cardiaque, et à n'appliquer aux moyens extracteurs qu'une telle séquence d'échantillons.

8. Le dispositif de la revendication 7, dans lequel les moyens sélecteurs sont des moyens aptes à calculer les intervalles RR desdits *B* battements consécutifs, et à définir un rythme cardiaque stable pour cette séquence d'échantillons si aucun de ces intervalles ne varie de plus d'un pourcentage donné, notamment pas plus de 10 %, par rapport à la valeur moyenne de l'intervalle RR calculée sur les *B* battements.

**Patentansprüche**

1. Vorrichtung zur Analyse von Alternationen von Zyklus zu Zyklus und/oder der Veränderlichkeit der Welle der ventrikulären Repolarisation in einem EKG-Signal, welches eine Reihe von Herzschlägen enthält, wobei dieses Signal zuvor durch eine medizinische Vorrichtung gesummelt wurde, danach gefiltert, abgetastet und digitalisiert, wobei diese Vorrichtung zur Analyse **gekennzeichnet ist durch**:

   - Extraktionsmittel, angepasst aus dem EKG-Signal, für jeden Herzschlag, ein zeitliches Segment von T Proben der Welle der ventrikulären Repolarisation zu extrahieren, betrachtet ausgehend von einem Zeitpunkt *t*, welcher einem vorherbestimmten zeitlichen Ursprung folgt;

- Speichermittel, angepasst, die so extrahierten T Proben zu speichern, für *B* aufeinander folgende Schläge, die betrachtet werden ausgehend vom *b-ten* Schlag des EKG-Signals, um ein zweidimensionales Aggregat $(C_{t,b}^{Tm,B})$ von *T x B* Schlägen des Signals auszuwählen und zu speichern, aneinander grenzend im Zeit-Schläge-Raum;

- Evaluationsmittel, angepasst, einen Faktor der lokalen Varianz der Welle der ventrikulären Repolarisation (Var $(C_{t,b}^{Tm,B})$) zu berechnen, der repräsentativ für ein Maß der Varianz des Signalniveaus der Proben im Inneren des Aggregats ist; und

- Bewertungsmittel, angepasst dazu:

  ■ Alternierungen von Zyklus zu Zyklus der Welle der ventrikulären Repolarisation auf den *B* Schlägen des Aggregats zu erkennen und zu registrieren;

  • den Faktor der lokalen Varianz (Var($C_{t,b}^{Tm,B}$)) mit einem Faktor der lokalen Alternierung *(W($C_{t,b}^{T,B}$))* zu gewichten, der zwischen einem Minimum (W = 0), das der vollständigen Abwesenheit der Erkennung einer Alternierung auf den Schlägen des Aggregats entspricht, und einem Maximum (W = 1), welches der Erkennung einer permanenten und wiederkehrenden Alternierung auf allen Schlägen des Aggregats entspricht, liegt, und

  • einen Kennwert der Alternierung und der Veränderbarkeit (TVar$_b$) an einem Ausgang abzugeben, der, für einen gegebenen Schlag, abhängig vom Faktor der lokalen Varianz ist, gewichtet mit dem Faktor der lokalen Alternierung.

2. Vorrichtung nach Anspruch 1, worin die Bewertungsmittel Mittel sind, die dazu eingerichtet sind:

   a) den Faktor der lokalen Alternierung (W($C_{t,b}^{T,B}$)) zu berechnen für eine vorherbestimmte Vielzahl zeitlicher Untersegmente von unterschiedlichen Dauern und Ursprüngen inmitten des Aggregats, danach
   b) unter diesen Untersegmenten diejenigen ($T_m$, $t_0$) auszuwählen, für welche der entsprechende Faktor der lokalen Alternierung *(W($C_{t0,b}^{Tm,B}$))* maximal ist, und
   c) den Faktor der lokalen Varianz (Var ($C_{t0,b}^{Tm,B}$) ), welcher dem so ausgewählten Untersegment entspricht, mit dem Faktor der maximalen lokalen Alternierung Max$_{Tm,t0}$ (W($C_{t,b}^{T,B}$)) zu gewichten.

3. Vorrichtung nach Anspruch 1 oder 2, in welcher die Gewichtung mit dem Faktor der lokalen Alternierung angewandt wird auf die Quadratwurzel des Faktors der lokalen Varianz

$$(\sqrt{Var(C_{t,b}^{Tm,B})}).$$

4. Vorrichtung nach Anspruch 1, in welcher die Bewertungsmittel Mittel sind, die dazu angepasst sind, den Faktor der lokalen Alternierung durch Suchen und Quantifizieren der Wiederholung eines vorher definierten Alternierungsmusters im Inneren des Aggregats zu berechnen, für eine gleiche Position der Proben in dem zeitlichen Segment.

5. Vorrichtung nach Anspruch 4, in welcher die Bewertungsmittel Mittel sind, die dazu angepasst sind, den Faktor der lokalen Alternierung durch Suchen und Registrieren von aufeinander folgenden Vorzeichenänderungen der Ableitung des Signals in der Domäne der Schläge zu berechnen, für eine gleiche Position der Schläge in dem zeitlichen Segment.

6. Vorrichtung nach Anspruch 1, welche weiterhin Mittel zur Vorbehandlung des Signals umfasst, vor dessen Anwendung auf die Extraktionsmittel, wobei diese Mittel zur Vorbehandlung umfassen wenigstens eines der Mittel der Gruppe, welche umfasst: ein Tiefpassfilter (14); ein Tiefpassfilter mit endlicher Impulsantwort (14); ein Filter zur Eliminierung der isoelektrischen Linie (16); und ein Filter zur Eliminierung der Atmungskomponente (18).

7. Vorrichtung nach Anspruch 1, weiterhin umfassend Mittel zur Auswahl von Schlägen (12, 20, 22), eingerichtet, in dem EKG-Signal eine Sequenz von Schlägen zu suchen, welche über die gesamte Dauer von *B* aufeinander folgenden Schlägen einen stabilen Herzrhythmus vorweist und nur Zyklen umfasst, die von sinusalem Ursprung sind, vorbehaltlich die Zyklen, die durch einen Herzschrittmacher hervorgerufen werden, und dazu, auf die Extraktionsmittel nur eine solche Sequenz von Schlägen anzuwenden.

8. Vorrichtung nach Anspruch 7, in welcher die Selektionsmittel Mittel sind, die dazu eingerichtet sind, die Intervalle RR der *B* aufeinander folgenden Schläge zu berechnen, und einen stabilen Herzrhythmus für diese Sequenz von Schlägen zu definieren, wenn keines dieser Intervalle sich um mehr als einen gegebenen Prozentsatz unterscheidet, insbesondere nicht mehr als 10%, im Verhältnis zum Mittelwert des Intervalls RR, das für die *B* Schläge berechnet wurde.

**Claims**

1. A device for the analysis of the cycle-to-cycle alternans and/or the variability of the ventricular repolarisation wave in an ECG signal including a series of cardiac beats, said signal having been previously collected by a medical device then filtered, sampled and digitized,
   said analysis device being **characterised by** :

   - extracting means, adapted to extract from the ECG signal, for each cardiac beat, a time segment of T samples of the ventricular repolarisation wave, as considered from a moment *t* which follows a predetermined time origin;
   - memorizing means, adapted to memorize the thus extracted T samples, for *B* consecutive beats as considered from the *b*th beat of the ECG signal, so as to select and memorize a two-dimensional aggregate ($C_{t,b}^{Tm,B}$) of *T*x*B* contiguous signal samples in the "time-beats" space;
   - evaluating means, adapted to calculate a local variance factor of the ventricular repolarisation wave(Var($C_{t,b}^{Tm,B}$)), representative of a measurement of the variance of the signal level in the samples within the aggregate; and
   - weighting means, adapted to:

     · detect and count the cycle-to-cycle alternans of the ventricular repolarization wave on the *B* beats of the aggregate,
     · weight the local variance factor (Var($C_{t,b}^{Tm,B}$)) by a local alternans factor (W($C_{t,b}^{T,B}$)) varying between a minimum (W = 0) corresponding to a complete absence of detection of an alternans over the beats of the aggregate, and a maximum (W = 1) corresponding to the detection of a permanent and recurring alternans over all the beats of the aggregate, and
     · outputting an alternans and variability index (TVar$_b$) which is a function, for a given beat, of the local variance factor weighted by the local alternans factor.

2. The device of claim 1, wherein said weighting means are means adapted to:

   a) calculate the local alternans factor (W($C_{t,b}^{T,B}$)) for a predetermined plurality of time sub-segments of different durations and origins within said aggregate;
   b) select among said sub-segments the one (T$_m$, to) for which the corresponding local alternans factor (W($C_{to,b}^{Tm,B}$)) is at a maximum; and
   c) weighting the local variance factor (Var($C_{to,b}^{Tm,B}$)) corresponding to the thus selected sub-segment, by said maximum local alternans factor *(Max$_{Tm,to}$ (W($C_{t,b}^{T,B}$))).

3. The device of claim 1 or 2, wherein the weighting by the alternans factor further is applied to the square root of the local variance factor (Var($C_{t,b}^{Tm,B}$)).

4. The device of claim 1, wherein the weighting means are means adapted to calculate the local alternans factor by searching and quantifying the repetition of a preset alternans pattern within the aggregate, for a same position of the sample in the time segment.

5. The device of claim 4 wherein the weighting means are means adapted to calculate the local alternans factor by searching and counting the consecutive changes of sign of the derivative of the signal in the beat domain, for a same position of the sample in the time segment.

6. The device of claim 1, further comprising means for pre-treating the signal before its application to the extracting means, said pre-treating means including at least one of the means of the group including: a low-pass filter (14); a low-pass filter with a finite pulse response (14); a filter for the elimination of the isoelectric line (16); and a filter for the elimination of the respiratory component (18).

7. The device of claim 1, further comprising sample selecting means (12, 20, 22), adapted to search in the ECG signal a sequence of beats presenting, over the whole duration of the $B$ consecutive beats, a cardiac rhythm which is stable and includes only cycles of sinusal origin but the cycles generated by a cardiac pacemaker, and to only apply to the extracting means such a sequence of samples.

8. The device of claim 7, wherein the selecting means are means adapted to calculate the RR intervals of said $B$ consecutive beats, and to define a stable cardiac rhythm for this sequence of samples if none of said intervals varies by more than a given percentage, in particular not more than 10%, compared to the average value of the RR interval calculated over the $B$ beats.

10 — Recueil et mémorisation du signal ECG

12 — Détection des pics de l'onde R

14 — Préfiltrage de l'ECG (filtre FIR passe-bas)

16 — Elimination de la ligne isoélectrique

18 — Elimination des composantes respiratoires

20 — Calcul de l'intervalle RR

22 — Test de stabilité du rythme (±10%)

24 — Sélection de la dérivation

26 — Extraction du segment de repolarisation
Paramétrage manuel de la largeur du QRS
et de la longueur du segment de repolarisation
(avec moyenne sur l'intervalle)

28 — Alignement des segments de repolarisation

30 — Balayage pour recherche de l'intervalle
optimal de repolarisation pour la
quantification de l'indice TVAR

32 — Calcul de l'indice TVAR

34 — Affichage

Figure 1

Figure 2

Niveau du
signal
(µV)

$C^{20,16}_{to,b}$

Figure 3a

Niveau du
signal
(µV)

Figure 3b

Figure 4a

Temps
(échantillons)

Figure 4b

Temps
(échantillons)

**Figure 5a**

**Figure 5b**

Figure 6a

Figure 6b

Figure 7a

Figure 7b